# EUROPEAN PATENT SPECIFICATION

(11) **EP 3 388 831 B1**
(45) Date of publication and mention of the grant of the patent: **30.12.2020**
(21) Application number: 16872762.6
(22) Date of filing: 14.11.2016
(51) Int. Cl.: G01N 33/00, G01N 29/02

(54) **FUEL OIL IDENTIFICATION USING A SURFACE STRESS SENSOR COATED WITH HYDROCARBON-GROUP-MODIFIED NANOPARTICLES**
KRAFTSTOFFERKENNUNG MIT EINEM OBERFLÄCHENBELASTUNGSSENSOR BESCHICHTET MIT KOHLENWASSERSTOFFMODIFIZIERTEN NANOPARTIKELN
IDENTIFICATION DU CARBURANT UTILISANT UN CAPTEUR DE CONTRAINTE DE SURFACE REVÊTUS DE NANOPARTICULES MODIFIÉES PAR DES GROUPES HYDROCARBURE

(30) Priority: 08.12.2015 JP 2015239115
(43) Date of publication of application: 17.10.2018
(73) Proprietor: National Institute for Materials Science, Tsukuba-shi, Ibaraki 305-0047 (JP)
(72) Inventor: SHIBA, Kota, Tsukuba-shi, Ibaraki 305-0047 (JP); YOSHIKAWA, Genki, Tsukuba-shi, Ibaraki 305-0047 (JP); IMAMURA, Gaku, Tsukuba-shi, Ibaraki 305-0047 (JP)
(74) Representative: Dehns
(86) International application number: PCT/JP2016/083689
(87) International publication number: WO 2017/098862

(56) References cited:
- WO-A1-2016/121155
- JP-A- H08 282 795
- JP-A- H08 327 576
- JP-A- H08 505 944
- JP-A- H09 243 630
- JP-A- H09 305 567
- JP-A- 2002 357 538
- JP-A- 2006 511 800
- JP-A- 2011 529 575
- JP-A- 2014 506 613
- US-A- 6 165 347
- HADI ALQAHTANI ET AL: "Highly sensitive alkane odour sensors based on functionalised gold nanoparticles", SENSORS AND ACTUATORS B: CHEMICAL, ELSEVIER BV, NL, vol. 160, no. 1, 30 July 2011 (2011-07-30) , pages 399-404, XP028110916, ISSN: 0925-4005, DOI: 10.1016/J.SNB.2011.07.068 [retrieved on 2011-08-06]
- JISUN IM ET AL: "A hybrid chemiresistive sensor system for the detection of organic vapors", SENSORS AND ACTUATORS B: CHEMICAL, ELSEVIER BV, NL, vol. 156, no. 2, 14 February 2011 (2011-02-14), pages 715-722, XP028228681, ISSN: 0925-4005, DOI: 10.1016/J.SNB.2011.02.025 [retrieved on 2011-03-01]
- YVONNE JOSEPH ET AL: "Gold-nanoparticle/organic linker films: self-assembly, electronic and structural characterisation, composition and vapour sensitivity", FARADAY DISCUSSIONS, vol. 125, 11 August 2003 (2003-08-11), pages 77-97, XP055604774, ISSN: 1359-6640, DOI: 10.1039/B302678G
- COOPER JAMES SCOTT: "Gold Nanoparticle Chemiresistor Sensor Array that Differentiates between Hydrocarbon Fuels Dissolved in Artificial Seawater", Anal Chem, vol. 82, no. 9, 2010, pages 3788-3795, XP055506583,
- PARK DEOG-SU: "The electrochemical sensor for methanol detection using silicon epoxy coated platinum nanoparticles", Sens Actuators B Chem, vol. 174, 2012, pages 45-50, XP028953234,
- FREUND M S: "A chemically diverse conducting polymer-based ''electronic nose", Proc Natl Acad Sci USA, vol. 92, no. 7, 1995, pages 2652-2656, XP000572385,
- GUO HUIHUI: "A Room Temperature Polymer-Coated Piezoresistive Silicon Bridge Gasoline Vapor Sensor", IEEE Sens J, vol. 12, no. 5-6, 2012, pages 926-929, XP011440764,
- GENKI YOSHIKAWA: "Nanomechanical Membrane-Type Surface Stress Sensor (MSS)", Journal of the Surface Science Society of Japan, vol. 35, no. 10, 2014, pages 571-576, XP055547492,
- FREDERIC LOIZEAU ET AL: "Piezoresistive membrane-type surface stress sensor arranged in arrays for cancer diagnosis through breath analysis", MICRO ELECTRO MECHANICAL SYSTEMS (MEMS), 2013 IEEE 26TH INTERNATIONAL CONFERENCE ON, IEEE, 20 January 2013 (2013-01-20), pages 621-624, XP032339313, DOI: 10.1109/MEMSYS.2013.6474318 ISBN: 978-1-4673-5654-1
- Frédéric Loizeau ET AL: "Membrane-Type Surface Stress Sensor with Piezoresistive Readout", Procedia Engineering, vol. 47, 1 January 2012 (2012-01-01), pages 1085-1088, XP55645374, NL ISSN: 1877-7058, DOI: 10.1016/j.proeng.2012.09.339

## Description

### Technical Field

The present invention relates to a fuel oil identification method using a sensor coated with particles which are modified with hydrocarbon groups such as alkyl groups or aryl groups.

### Background Art

Identification among mixtures of gases including a plurality of components is a key issue in various applications from medical care to a variety of industries. As an example, identification among fuel oils has been developed since it is a technique relating to various industries such as an automobile industry concerning specification of gasoline diluted with impurities. Many of the conventional techniques include qualitative analysis of the total components by gas chromatography and quantitative analysis by a mass spectrometer. In such methods, identification cannot be conducted conveniently because the methods require large and expensive dedicated equipment. To address this issue, identification techniques using a small sensor have been developed. The identification using a sensor employs a pattern recognition technique. That is, the surfaces of a plurality of sensors are respectively coated with receptor materials with different properties and a "sensor array" in which those sensors are arranged in an array is used for the measurement to obtain different responses from the respective sensors so as to conduct the pattern recognition. This technique would be able to be applied to various mixed gases, and could be one of the effective approaches for the identification. This technique would, however, require to prepare a relatively large number of materials with different properties and to coat the surfaces of the sensors with them, and thus it would not be easy to optimize the coating conditions for each material. Further, as in the case of substances like fuel oils such as regular gasoline (hereinafter, sometimes abbreviated to "regular gas") or high-octane gasoline (hereinafter, sometimes abbreviated to "high-octane" or "high-octane gas") in which their main components are similar and ratios among them are slightly different, most of the sensors in the sensor array show similar responses, which might make the identification difficult. Thus, it is required to develop a technique which can achieve highly accurate identification more easily and simply.

Non Patent Literature 1 discloses a sensor in which a surface of a silicon substrate is coated with silicone rubber, and a silicone rubber membrane is swelled by absorbing gasoline vapor to detect changes in surface stress applied on the silicon substrate. However, such type of sensor has limitation in application because silicone rubber slowly absorbs and releases gasoline vapor, and thus response speed is slow. Further, the literature specifically discloses detection of gasoline, and provides no description of identification with respect to multiple types of fuel oils. Non Patent Literature 3 discloses a sensor sensitive to alkane odours (e.g. petrol vapours), comprising a glass substrate with gold electrodes and coated with a receptor layer, which comprises gold nanoparticles modified with dodecyl groups. Patent Literature 2 is an intermediate publication, disclosing a membrane type surface stress sensor coated with nanoparticles that are modified with hydrocarbon groups.

### Summary of Invention

### Technical Problem

An object of the present invention is to provide a method of fuel oil identification which uses a sensor which enables highly accurate identification of fuel oils by using a material which has high compatibility with a main component of fuel oils as a receptor layer material.

### Solution to Problem

The present invention provides a fuel oil identification method comprising:
supplying vapor of an analyte to a fuel oil identification sensor; and
identifying the analyte on the basis of an output of the fuel oil identification sensor
wherein the fuel oil identification sensor comprises:
   a surface stress sensor, and
   a receptor layer comprising particles of 1 mm or less in particle size and modified with hydrocarbon groups, the receptor layer coating a surface of the surface stress sensor, and
   wherein said surface stress sensor is adapted to detect a change in surface stress when said receptor layer is contacted by vapor of said fuel oil.

The particles may be nanoparticles having a particle size of 100 nm or less.

Further, the particles may include: zeolite; Metal-Organic Frameworks; a hybrid of an oxide and/or a metal, and various surfactants; a porous material; graphene; an alkali silicate or a titanate such as a clay mineral; and a low-dimensional compound including a transition metal oxoate.

Further, the receptor layer may further include a matrix material other than the particles.

Further, the hydrocarbon group may be an alkyl group or an aryl group.

Further, the alkyl group may be an alkyl group having 1 to 30 carbon atoms.

Further, the alkyl group may be a dodecyl group or an octadecyl group.

Further, the fuel oil may be a fuel oil selected from the group consisting of regular gasoline; high-octane gasoline; diesel oil; heating oil; a mixture of regular gasoline or high-octane gasoline, and an alcohol; heavy oil A; a mixture of heavy oil A and heating oil; and a mixture of at least one of heavy oil A and heating oil, and diesel oil.

Here, a mixture of the vapor of the analyte and another gas may be supplied to the fuel oil identification sensor.

Further, the other gas may be a gas or a mixture of a plurality of gases selected from the group consisting of nitrogen, air, argon, and helium.

Further, the vapor of the analyte may be generated without performing heating or cooling.

Further, the vapor of the analyte may be generated at room temperature.

Further, the vapor of the analyte may be supplied to different types of fuel oil identification sensors; and the analyte may be identified on the basis of an output obtained from each of the fuel oil identification sensors.

Further, vapors of a plurality of analytes may be switched in sequence for delivery to the fuel oil identification sensor

Cleaning of the fuel oil identification sensor may not be performed for at least one cycle of the switching for the delivery.

Further, identification of the analyte may be performed by further information processing of the output of the fuel oil identification sensor.

Here, the information processing may be based on at least one analytical method selected from principal component analysis, a neural network, deep learning, a support vector machine, random forest, a decision tree, regression analysis, and big data analysis.

### Advantageous Effects of Invention

According to the present invention, use of hydrocarbon-group-modified particles as a receptor layer enables identification of fuel oils by a single sensor, which has been difficult by conventional identification sensors. Accordingly, situations requiring multiple sensors, such as a conventional sensor array, to which different receptor materials are coated are reduced, resulting in significant simplification of factors in a sensor production step, a sensor system, and data analysis. As described below, the present invention does not, of course, exclude use of multiple sensors such as a sensor array, and it is of course possible to apply a form of the sensor array or the like, as required.

### Brief Description of Drawings

Fig. 1 is a conceptual diagram showing an example of a configuration of devices utilized for synthesis of octadecyl group-modified nanoparticles.
Fig. 2 is a scanning electron microscope (SEM) image of octadecyl group-modified nanoparticles.
Fig. 3 is an optical microscope image of a membrane-type surface stress sensor before and after coating with octadecyl group-modified nanoparticles.
Fig. 4A shows results of measurements of 23 types of compounds by using an octadecyl group-modified nanoparticles-coated membrane-type surface stress sensor (results with respect to 12 types among the 23 types of compounds are shown).
Fig. 4B shows results of measurements of 23 types of compounds by using an octadecyl group-modified nanoparticles-coated membrane-type surface stress sensor (results with respect to 11 types among the 23 types of compound are shown).
Fig. 5 shows relative signal intensities in which signal intensities with respect to vapors of samples are divided by a signal intensity with respect to water vapor shown in Fig. 4A and Fig. 4B.
Fig. 6 shows results of measurements of seven types of alkanes by using an octadecyl group-modified nanoparticles-coated membrane-type surface stress sensor.
Fig. 7A shows results of measurements of seven types of alkanes by using PVP-coated membrane-type surface stress sensor.
Fig. 7B shows results of measurements of seven types of alkanes by using PAA-coated membrane-type surface stress sensor.
Fig. 7C shows results of measurements of seven types of alkanes by using PMMA-coated membrane-type surface stress sensor.
Fig. 8 shows results of principal component analysis on the basis of the results of the measurements of seven types of alkanes by using octadecyl group-modified nanoparticles-, PVP-, PAA-, and PMMA-coated membrane-type surface stress sensors.
Fig. 9A is a magnified view of a PVP part in Fig. 8.
Fig. 9B is a magnified view of a PAA part in Fig. 8.
Fig. 9C is a magnified view of a PMMA part in Fig. 8.
Fig. 10 shows results of measurements of regular gasoline, high-octane gasoline, diesel oil, heating oil, and regular gasoline mixed with heating oil by using an octadecyl group-modified nanoparticles-coated membrane-type surface stress sensor.
Fig. 11A shows results of measurements of regular gasoline high-octane gasoline, diesel oil, heating oil, and a mixture of regular gasoline and heating oil by using a PVP-coated membrane-type surface stress sensor.
Fig. 11B shows results of measurements of regular gasoline, high-octane gasoline, diesel oil, heating oil, and a mixture of regular gasoline and heating oil by using a PAA-coated membrane-type surface stress sensor.
Fig. 11C shows results of measurements of regular gasoline, high-octane gasoline, diesel oil, heating oil, and a mixture of regular gasoline and heating oil by using a PMMA-coated membrane-type surface stress sensor.
Fig. 12 shows results of principal component analysis on the basis of the results of the measurements of regular gasoline, high-octane gasoline, diesel oil, heating oil, and regular gasoline mixed with heating oil by using an octadecyl group-modified nanoparticles-, PVP-, PAA-, and PMMA-coated membrane-type surface stress sensor.
Fig. 13A is a magnified view of a PVP part in Fig. 12.
Fig. 13B is a magnified view of a PAA part in Fig. 12.
Fig. 13C is a magnified view of a PMMA part in Fig. 12.
Fig. 14A shows results of measurements of 23 types of compounds by using a dodecyl group modified a nanoparticles-coated membrane-type surface stress sensor (results with respect to 12 types among the 23 types of compounds are shown).
Fig. 14B shows results of measurements of 23 types of compounds by using a dodecyl group modified a nanoparticles-coated membrane-type surface stress sensor (results with respect to 11 types among the 23 types of compounds are shown).
Fig. 15 shows relative signal intensities in which signal intensities with respect to vapors of samples are divided by a signal intensity with respect to water vapor in Fig. 14A and Fig. 14B.

### Description of Embodiments

In the method of the present invention, a sensor with following features is used; a sensor coated with an alkyl group modified particles receptor layer constituted by direct deposition of hydrocarbon-group-modified particles on a sensor body which detects surface stress.

Here, the hydrocarbon groups which modify the particles include various types of hydrocarbon groups such as an alkyl group and an aryl group. When the hydrocarbon group is an alkyl group, a methyl group having one carbon atom to a triacontyl group having 30 carbon atoms can be preferably used. Although such an alkyl group exemplified in examples consists of a straight alkyl chain, the alkyl group is not limited to a straight alkyl chain, and an alkyl group having a side chain can also be used. Further, an unsaturated hydrocarbon with a chain structure can be used as well as an alkyl group. More generally, the hydrocarbon groups can be aromatic hydrocarbon groups and other cyclic hydrocarbon groups.

Note that binders, which improve a property of forming particle aggregation or adhering to a sensor body, or other constituents can be added in addition to particles which bind to an analyte. For example, a matrix material for regulating adhesion to a sensor body or disperse/aggregation of particles can be used, for example, a polymer or the like can further be added to particles. It is also possible to increase compatibility with particles by coating of a self-assembled monolayer, that is, it is possible to enhance adhesion between nanoparticles and a surface of a sensor body through a self-assembled monolayer.

By adsorption of a substance to be detected in a fuel oil by a hydrocarbon-group-modified particles receptor layer coated onto the surface of the surface stress sensor, the surface stress sensor detects changes of a stress produced in the receptor layer and outputs a signal.

In the hydrocarbon-group-modified particles receptor layer used in the method of the present invention, the shapes of particles on which a hydrocarbon group is fixed can be isotropic or anisotropic. Materials constituting the particles may include an elementary substance such as a metal, a compound such as an oxide or a sulfide, a polymer, an inorganic-organic hybrid, or a bio-related material such as a protein. Specifically, the materials which can be used include, but are not limited to, zeolite, Metal-Organic Frameworks (MOF), a hybrid of an oxide and/or a metal and various surfactants, a porous material, graphene, an alkali silicate or titanate such as a clay mineral, and a low-dimensional compound including a transition metal oxoate.

Further, the size of the particles is preferably 1 mm or less because adhesive force is increased due to greater influence of van der Waals interaction than that of gravity or the like. The particle size is more preferably 100 µm or less, even more preferably 1 µm or less, still more preferably 100 nm or less. In any case, the particle size is not limited so long as the particles adhere to a surface of a sensor body by a kind of interaction. In one aspect, however, it is important that the particles are not easily detached from the surface of the sensor body.

Any coupling material having a hydrocarbon group in the molecular structure, such as silane, phosphonic acid, thiol, amines, or a surfactant can be used for fixing a hydrocarbon group.

Further, hydrocarbon-group-modified particles synthesized by any method can be used. Specifically, the method includes, but is not specifically limited to, a precipitation reaction in a homogeneous solution, a reaction in a pseudo-homogeneous system utilizing an emulsion, a gaseous phase reaction utilizing spray drying or pyrolysis, or a solid phase reaction such as ball milling.

Examples of the sensor body coated with a hydrocarbon-group-modified particles receptor layer include various types of surface stress sensors as described in Patent Literature 1, for example. However, a shape, a material, a size, or the like of the sensor body is not specifically limited, and any object can be used. Preferred examples include, for example, a slice-like component which is supported at one or multiple portions. In addition, sensor bodies having various forms, such as slice-like objects supported at two or more portions, for example, a double-supported beam, membranes, and the like can be adopted.

Methods for coating a surface of a sensor with a hydrocarbon-group-modified particles receptor include, but are not specifically limited to, dip coating, spray coating, spin coating, casting, and a coating using a doctor blade.

Note that, as used in the specification and claims in the present invention, "a fuel oil" refers to a hydrocarbon-based oil whose main component is a specific distillate of crude oil, which is used as fuel for an internal combustion engine or various fuels such as heating oil, or an oil which is about equal in properties to the specific distillate, such as a synthetic petroleum derived from coal. However, fuel oils identified by the method of the present invention include not only a hydrocarbon such as a distillate of crude oil, but also the hydrocarbon to which various additives are added. For example, commercially available gasoline or diesel oil for automobiles contains various additives for the purpose of, for example, improving its properties. Further, plant-derived alcohol, which is not included in an amount of carbon dioxide emissions under a treaty when it is burned, is added to some gasoline, and fuel oils of the present invention also include such a fuel oil containing an ingredient which is not derived from crude oil. Further, as a fuel for an internal combustion engine, for example, for a fuel for automobiles, only fuels which comply with industrial standards or which are permitted by laws such as the tax law can be used. However, illegal gasoline containing heating oil, or oil produced by illegally treating heavy oil A or heating oil or adulterated diesel oil produced by adding such oil produced by illegally treating heavy oil A or heating oil to diesel oil is sometimes distributed and used stealthily. Thus, whether fuel oils comply with a standard/law or not, the present invention encompasses all of the fuel oils circulated and used as various fuel oils.

Further, in examples described below, nitrogen was used as a carrier gas for sending sample vapor to a sensor chamber, or used to accelerate desorption of an adsorbed sample. However, it is of course not limited to nitrogen, and any gas including air, nitrogen, argon, helium, and a mixed gas containing two or more of these gases may be used. Although carrier gases are described above, sample vapor can be sent to a sensor together with a gas present in vacant spaces such as upper space of a container containing the sample without supplying any gas from outside as a carrier gas for sending the sample vapor to the sensor. Further, needless to say, vapor of a sample can be solely sent to a sensor without mixing with other gases. Alternatively, when a sensor is placed in a position where vapor from a sample can naturally arrive at by convection, diffusion or the like, for example, when a sensor is placed in a container (e.g., upper space of the container) such as a fuel tank containing the sample, or a sensor is placed in another area close and communicating to the container, even sending of vapor is not required.

Further, in examples, a sensor was cleaned by using nitrogen, then various sample vapors were allowed to flow for the measurements of sensor responses with respect to the samples to identify difference of the responses by principal component analysis. However, the method for measurements and analytical methods are of course not limited to the methods used in examples. For example, for identifying types of gasoline or presence or absence of mixed materials, a possible application method includes allowing one type of sample vapor to flow, and immediately allowing another type of sample vapor to flow without cleaning the sensor by using nitrogen or the like, and then measuring changes of sensor responses at that time. Further, any analytical approaches can be used including a neural network, deep learning, a support vector machine, random forest, a decision tree, and regression analysis, and a specific analytical method which is an application of a big data analysis technology.

Hydrocarbon-group-modified particles receptors used in the method of the present invention include the following characteristics.
(1) A surface of the particles is coated with hydrocarbon groups to achieve compatibility with a hydrocarbon-based compound, which is a main component of a fuel oil.
(2) The type of hydrocarbon compounds which can be detected or identified can be changed by changing structures of hydrocarbon groups which are immobilized on the surface of particles. Further, since materials which can be favorably detected/identified vary depending on immobilized hydrocarbon groups, by using a plurality of types of sensors of the present invention coated with particles modified with different types of hydrocarbon groups and combining outputs of the sensors, it is possible to detect/identify a wider variety of hydrocarbons, fuel oils, and the like.
(3) By using particles as a material of a receptor layer, space (a route) for a hydrocarbon-based compound to reach the inside of the receptor layer can be secured. Specifically, by changing sizes or shapes of stacked hydrocarbon-group-modified particles, sizes of voids formed in the hydrocarbon-group-modified particle can be controlled. The existence of the route communicating to the inside of the receptor layer provides higher responsivity as compared to a receptor layer, such as a conventional polymer layer, in which an analyte arrives at the inside of the receptor layer by mainly diffusion of the analyte from the surface of a membrane into the inside of the receptor layer.
(4) High water resistance is achieved by modification with a hydrophobic hydrocarbon group. For example, measurement can be performed even under high-humidity environments without influence of humidity.
(5) In the following examples, detection was performed by measuring analytes evaporated from a fuel oil at room temperature (about 25°C), whereas an analyte can of course be evaporated by heating when the analyte was supplied to a sensor of the present invention. Although gasoline is extremely volatile, since diesel oil whose boiling point is defined as about 350°C or less was also detected in the same manner as gasoline, it was demonstrated that a sensor of the present invention exhibited a high capability to detect/identify fuel oils, which are defined to have a high boiling point, around room temperature. With respect to the description using "room temperature", temperatures in practical application can be a considerably higher temperature or lower temperature than room temperature according to environments in which a fuel oil is placed, for example, when fuels are in a tank placed in a location susceptible to heat of an internal combustion engine or outside air temperature, or fuels are in piping through such a place. Even under such a condition, the present invention can be applicable without performing heating (or cooling) for detection.

### Examples

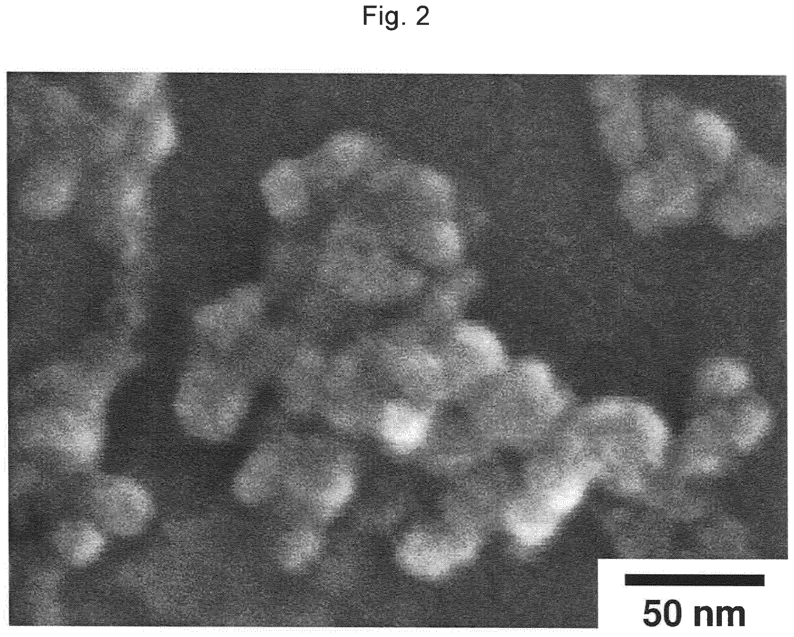

The present invention is described below in detail with reference to examples. It should be construed that the following examples are of course provided only to facilitate understanding of the present invention, and are not intended to limit the scope of the present invention to the examples in any way. In the following examples, although an example using an alkyl group as a hydrocarbon group is provided, the example is not intended to exclude hydrocarbon groups other than alkyl groups. Since nanoparticles shown in Fig. 2 were used in the following examples as any of the particles modified with hydrocarbon groups, the particles are referred to as the nanoparticles hereinbelow.

### <Example 1> Evaluation of properties of a surface stress sensor coated with octadecyl group-modified silica-titania hybrid nanoparticles

An example of preparation and measurement of a receptor layer using nanoparticles as described above is demonstrated.

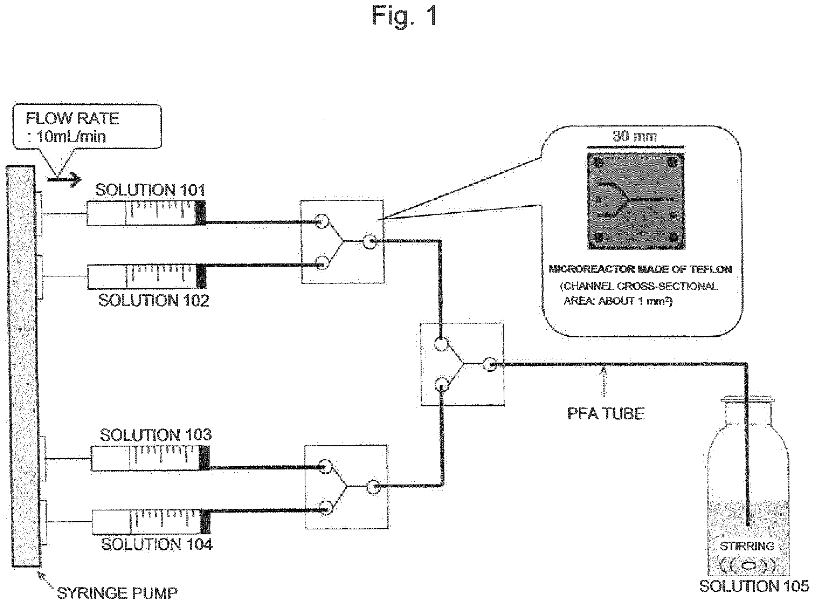

The above-mentioned nanoparticles were synthesized on the basis of a method shown in Non Patent Literature 2. Specifically, nanoparticles were synthesized by co-hydrolysis and a condensation polymerization reaction of octadecyl triethoxysilane (ODTES) and titanium tetraisopropoxide (TTIP) in an aqueous ammonia isopropanol (IPA) solution in which octadecylamine (ODA) was dissolved. The above-described synthesis reaction was performed using a microreactor having a micrometer-sized Y-shaped flow path made of Teflon (registered trademark) (Fig. 1). Four precursor solutions were prepared as follows: ODTES/IPA (solution 101), H₂O/IPA/ammonia (solution 102), TTIP/IPA (solution 103), and H₂O/IPA (solution 104), and volumes of the solution 101 through the solution 104 were equal to one another. Then, the precursor solutions were pumped concurrently at a constant speed using syringe pumps. The solution 101 and the solution 102, and the solution 103 and the solution 104 were respectively mixed in microreactors placed side by side, and then reaction solutions ejected from the two reactors were mixed in another microreactor to make one reaction solution. The reaction solution was added into a precursor solution, which was ODA/H₂O/IPA (Solution 105) prepared separately, and the mixture was stirred at a constant speed until the addition was completed. Then, the mixture was aged at room temperature to obtain the above-mentioned nanoparticle dispersion. Images of the nanoparticles observed by SEM are shown in Fig. 2.

The above-described nanoparticle dispersion was centrifuged to remove supernatant, and then IPA was added to the precipitate and the precipitate was redispersed by ultrasonication. The dispersion was centrifuged again, and the above-described step was further repeated twice to eliminate unreacted raw materials and the like. Then, the precipitate was dispersed in IPA, and sprayed on a sensor chip using a spray coater. A piezoresistive surface stress sensor having a membrane-type structure was used as a sensor.

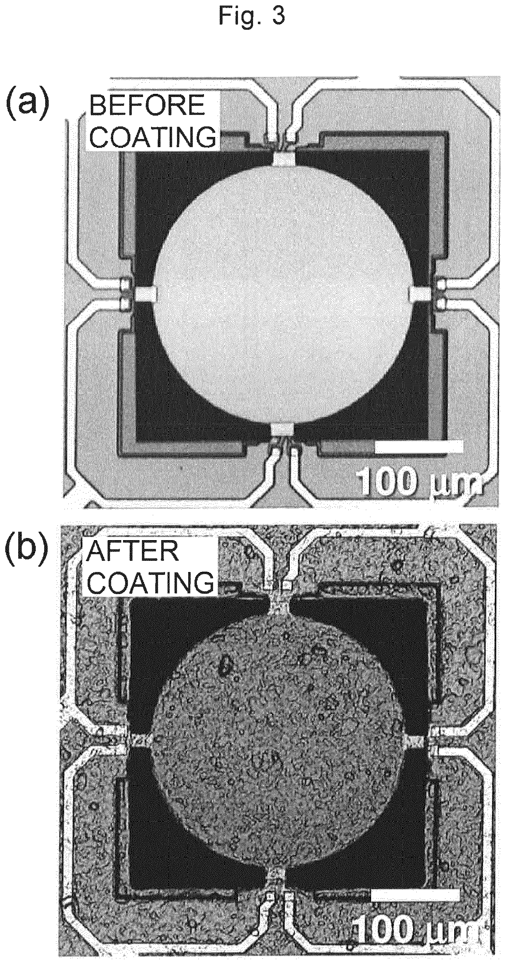

An optical microscope image of the sensor after the nanoparticle coating is shown in Fig. 3. It can be found that the whole membrane surface is coated as compared to that before the coating.

Subsequently, the following 23 types of chemical species were measured. Specifically, the 23 types of species used were water (ultrapure water), formaldehyde (formalin), acetic acid, acetone, methyl ethyl ketone (2-butanone), methanol, ethanol, IPA (isopropanol, 2-propanol), 1-butanol, 1-pentanol, n-hexane, n-heptane, n-octane, n-nonane, n-decane, n-undecane, n-dodecane, benzene, toluene, xylene (mixture of o-, m-, and p-xylene), 1,2-dichlorobenzene, 1,3-dichlorobenzene, and N,N-dimethylformamide. The above-described samples were dispensed in vials, nitrogen was supplied to the vials as a carrier gas at a flow rate of 100 mL/min regulated by a mass flow controller. Thus, the gas containing a certain amount of sample vapor collected in the headspace of the vial was introduced in a tightly-closed chamber in which the sensor was located. Here, the sample vapor was obtained at room temperature. Another mass flow controller was used and operated under the same conditions. However, this mass flow controller was connected with an empty vial, and then connected to the sensor chamber to introduce nitrogen containing no sample vapor. Accordingly, desorption of samples adsorbed on the receptor layer was accelerated to clean the receptor layer. The above-described sample introduction and cleaning were repeated at intervals of 30 seconds, and then results of the measurements were obtained.

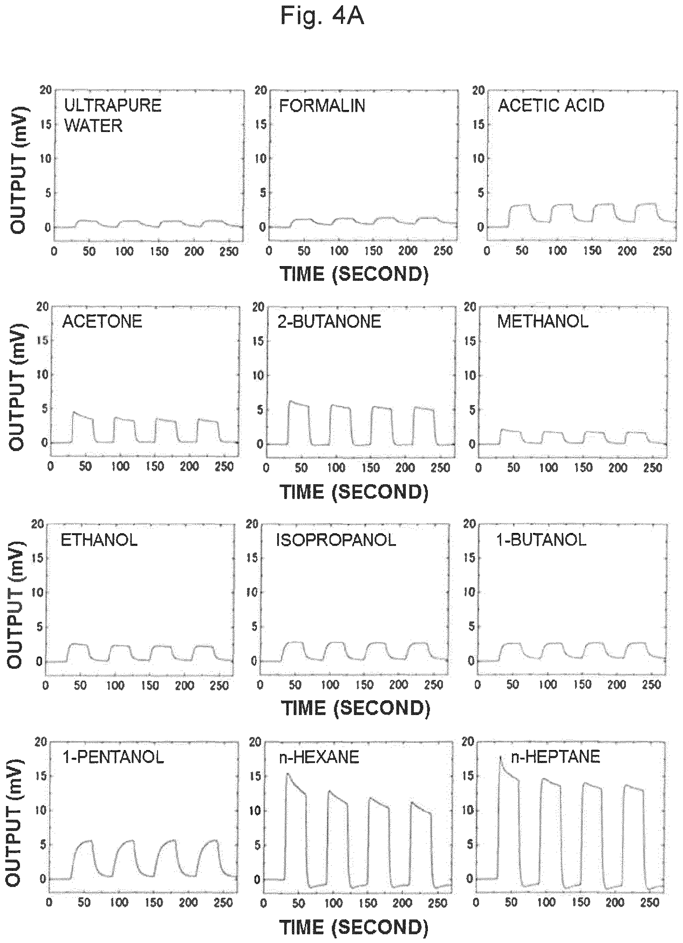

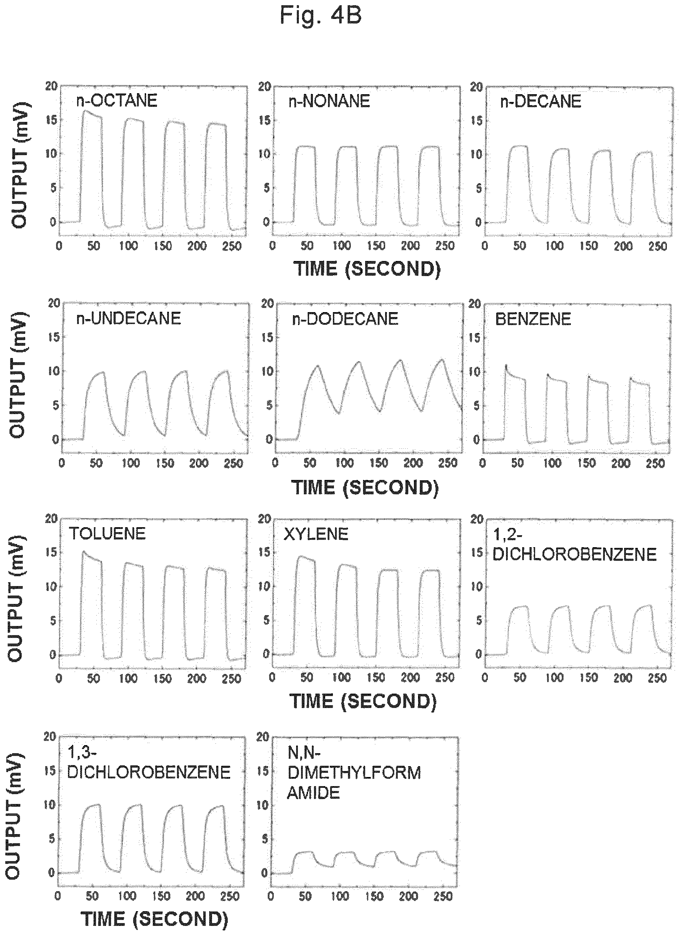

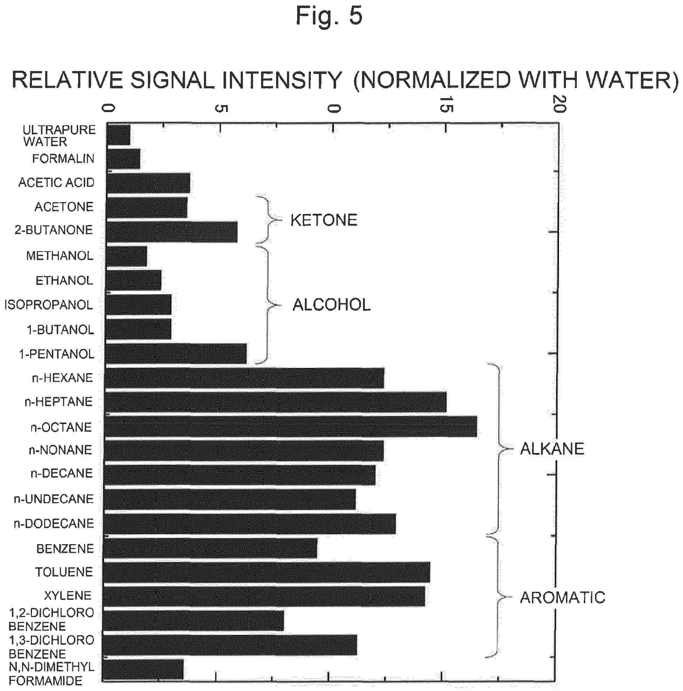

Results of the measurements of the sample vapors are shown in Fig. 4A and Fig. 4B. Further, relative signal intensities in which signal intensities with respect to the sample vapors are divided by a signal intensity with respect to water vapor are shown in Fig. 5. Responses with respect to compounds which are slightly soluble in water are larger than responses with respect to water soluble compounds. The findings can be explained by the fact that an octadecyl group is a hydrophobic functional group.

### <Example 2> An alkane identification capability of a surface stress sensor coated with octadecyl group-modified silica-titania hybrid nanoparticles

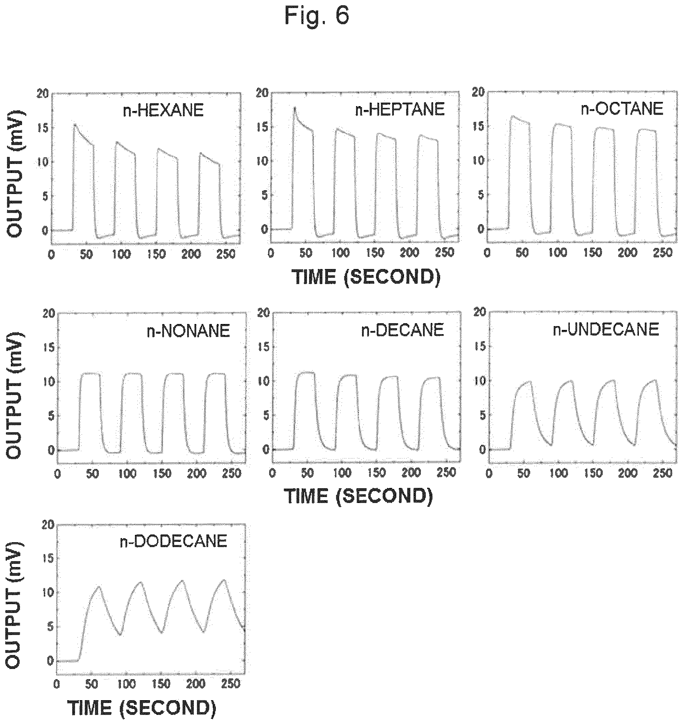

Next, capability of identification of a normal alkane molecule from other normal alkane molecules having very close chemical structures was studied by utilizing hydrophobic properties of the surface stress sensors coated with octadecyl group-modified silica-titania hybrid nanoparticles. As normal alkanes, seven types of alkanes having 6 to 12 carbon atoms were used. Here, results of the measurements are shown in Fig. 6. Sample vapors were obtained also at room temperature. It is found that each of the samples produced clearly different responses with respect to rising, intensity, and decay of signals. The difference is thought to reflect strengths of hydrophobic interactions between the normal alkane molecules, which were measured, and the octadecyl group which were stronger when lengths of alkyl chains constituting the alkane molecules were longer. With respect to decay part of the signals, longer alkyl chain length tends to result in decreased slope. The decreased slope means that the desorption of the alkane from the octadecyl group takes much time, supporting the above-described discussion.

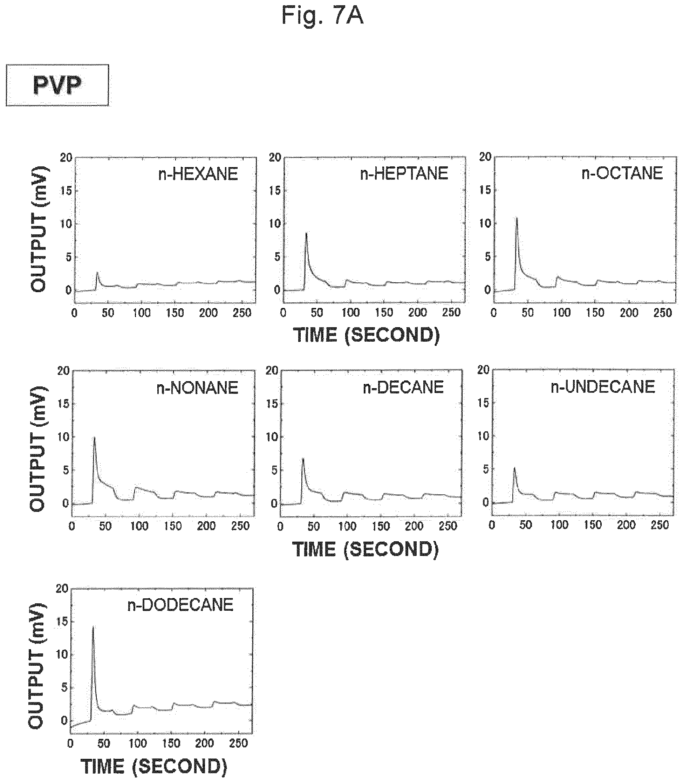

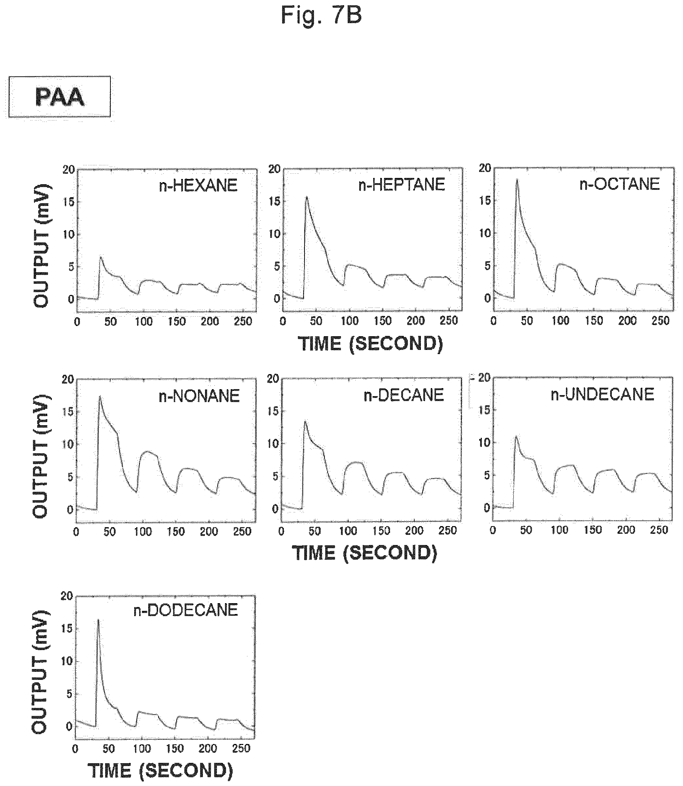

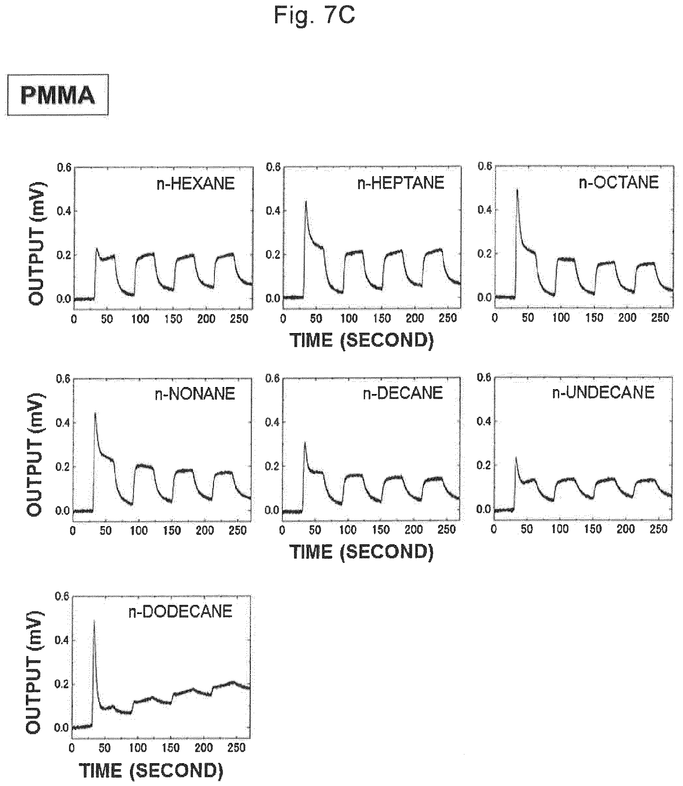

As comparative examples, three types of commercially available polymers (polyvinyl pyrrolidone: PVP, polyallylamine: PAA, and polymethyl methacrylate: PMMA) were used to perform measurements in a similar manner. Results of the measurements are shown in Fig. 7A to Fig. 7C. With respect to PVP and PAA, shapes of signals are changed with repeated measurements, and it is proved to be difficult to obtain consistent results. On the other hand, with respect to PMMA, although shapes of signals are relatively uniform, intensity is several tens of times smaller than that obtained by using octadecyl group-modified nanoparticles, and it is found to be inferior in detection sensitivity. Consequently, it was demonstrated that various alkanes can be measured with high sensitivity and high reproducibility by using octadecyl group-modified nanoparticles.

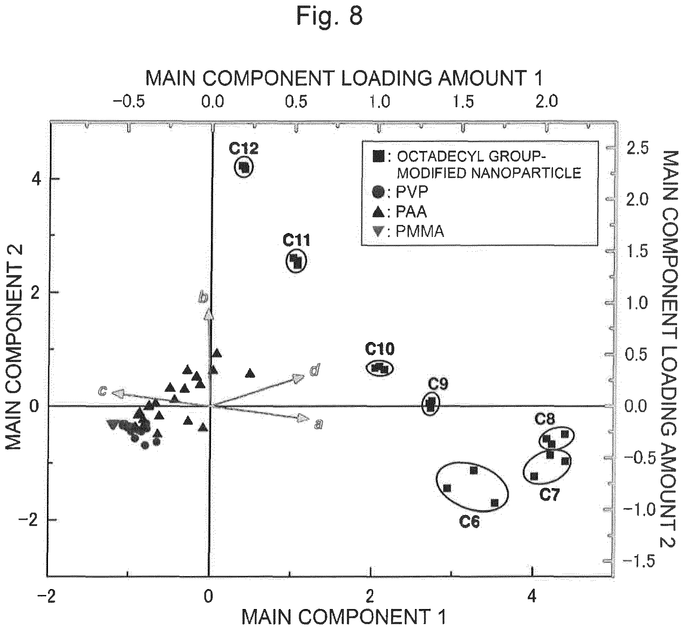

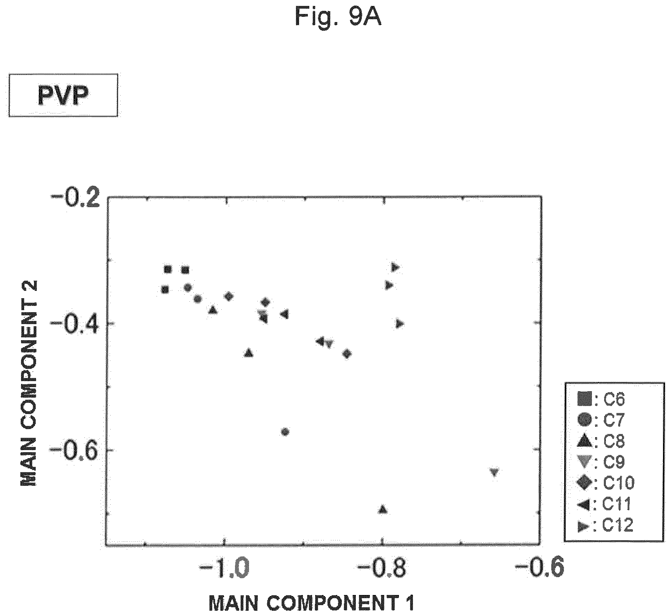

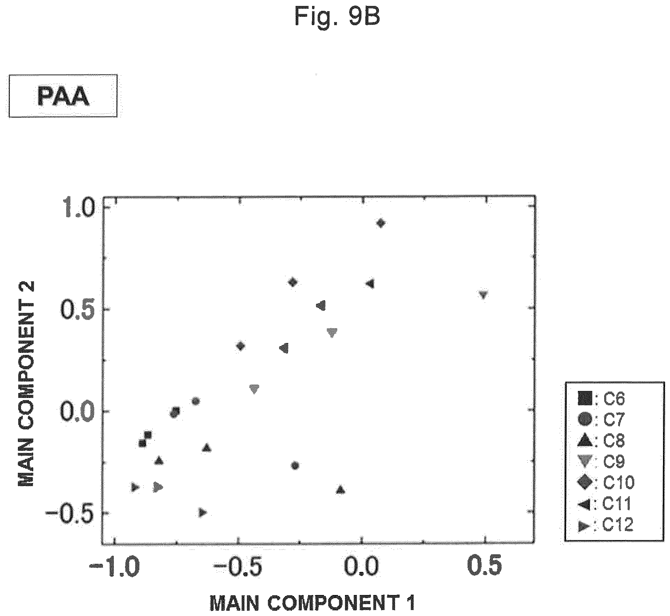

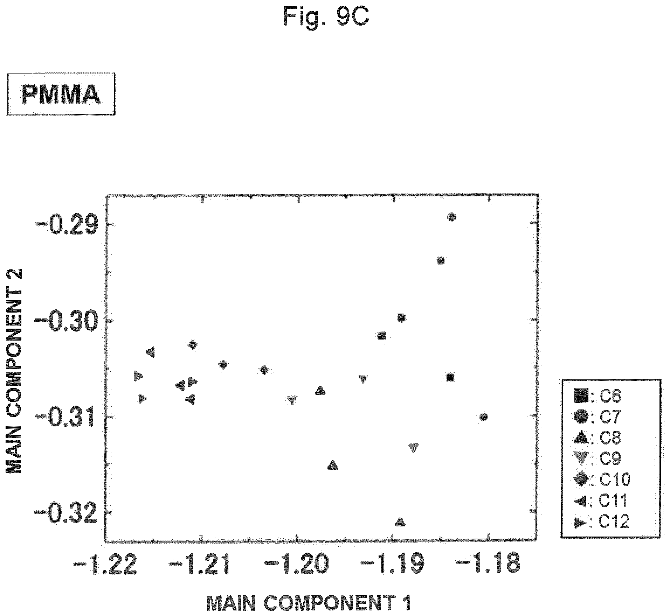

For quantitative interpretation of the above-described results of the measurements, principal component analysis was performed. Specifically, on the basis of results obtained from the measurements of seven types of alkanes by using octadecyl group-modified nanoparticles and three types of polymers, a slope of rising (0 to 5 seconds), a slope of midway (5 to 30 seconds), a slope of decay (30 to 35 seconds), and intensity at 5 seconds after the rising were extracted, and four-dimensional data sets were produced. From the results, four-dimensional mapping was performed, and a two-dimensional projection chart shown in Fig. 8 was obtained. Since parameters having different properties were used, all data were standardized in advance, and correlation matrix was used for calculation. Note that Origin 2015 from Lightstone Corp. was used as a software for the principal component analysis. When the octadecyl group-modified nanoparticles were used, the seven types of normal alkanes (the alkanes were identified by numbers of carbon atoms of each alkane appended to "C" in Fig. 8) were separated, and the results revealed that the alkanes can be identified. On the other hand, when the three types of polymers were used, principal component plots of the alkanes are clustered in small area, and identification is difficult. Magnified views of the areas around the plots with respect to the three types of polymers in Fig. 8 are shown in Fig. 9A to Fig. 9C, respectively. From the figures, it is also difficult to say that the seven types of the alkanes are separated with a certain tendency when measurements were performed by using the polymers, and thus it was demonstrated that the alkanes were hard to be identified.

### <Example 3> A fuel oil identification capability of a surface stress sensor coated with octadecyl group-modified silica-titania hybrid nanoparticles

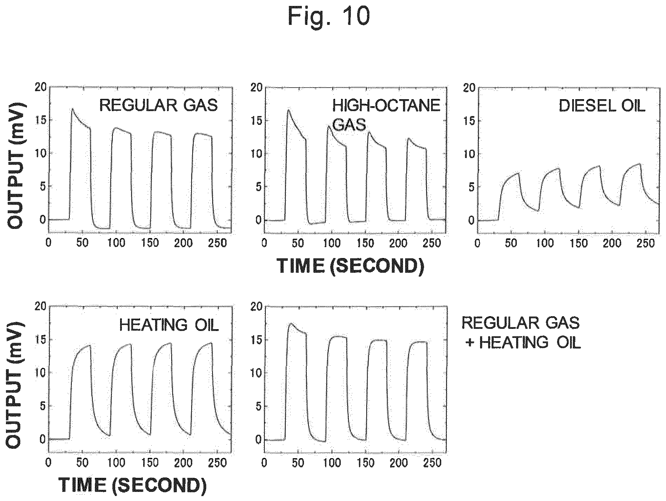

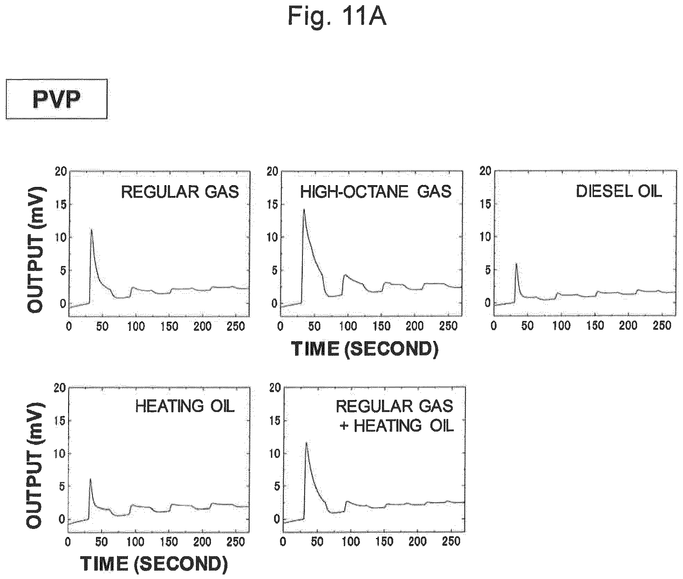

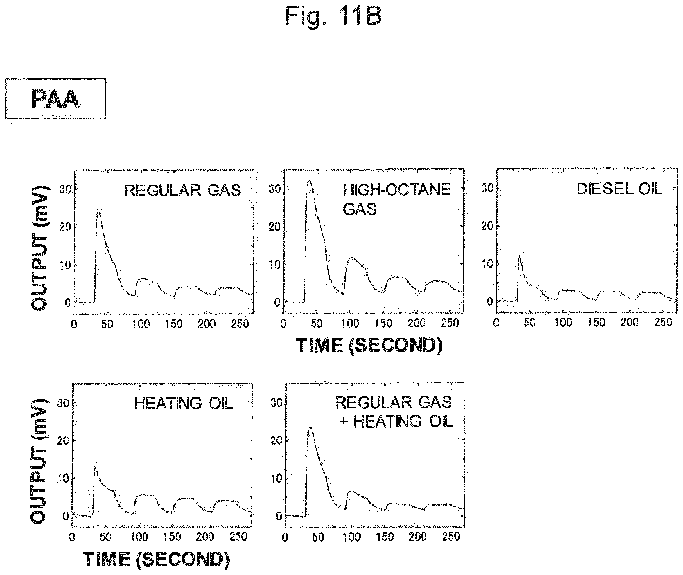

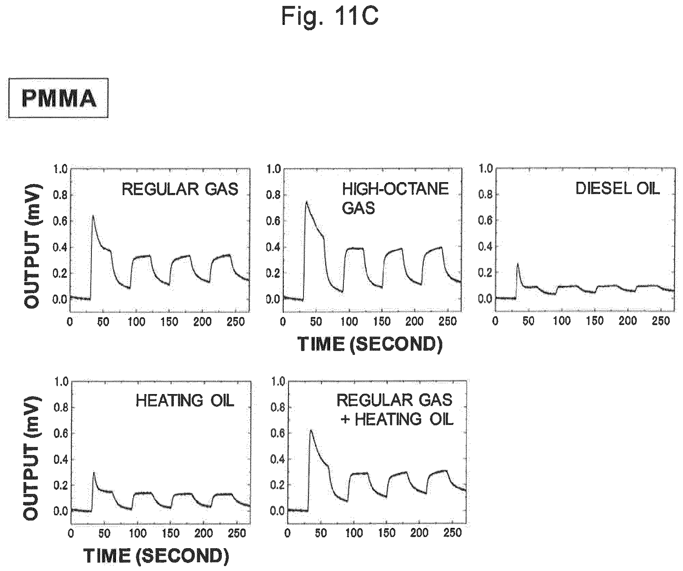

Measurements of four types of fuel oils were performed according to the method described in Example 1. Specifically, commercially available regular gasoline and high-octane gas, diesel oil which is commercially available as a fuel for diesel engine automobiles, and heating oil were used. Here, sample vapors were obtained also at room temperature. As receptor materials, the three types of polymers described in Example 2 were also used in addition to octadecyl group-modified silica-titania hybrid nanoparticles. Results of the measurements are shown in Fig. 10, and Fig. 11A to Fig. 11C. When the octadecyl group-modified nanoparticles were used, it is found that each of the samples produced clearly different response with respect to rising, intensity, and decay of signals similarly to the measurements of seven types of normal alkanes described in Example 2. Similarly, when the three types of polymers were used, shapes of signals are changed with repeated measurements with respect to PVP and PAA, and it is proved to be difficult to obtain consistent results. On the other hand, with respect to PMMA, although shapes of signals are relatively uniform, intensity is several tens of times smaller than that obtained by using octadecyl group-modified nanoparticles, and it is found to be inferior in detection sensitivity.

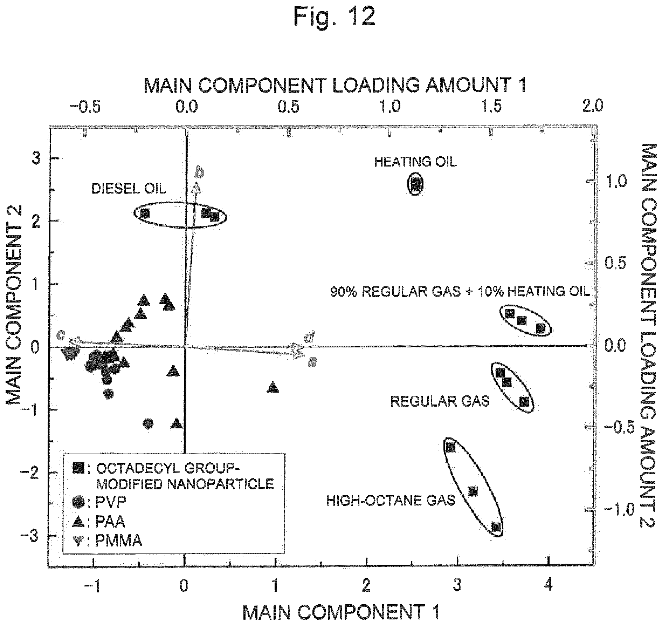

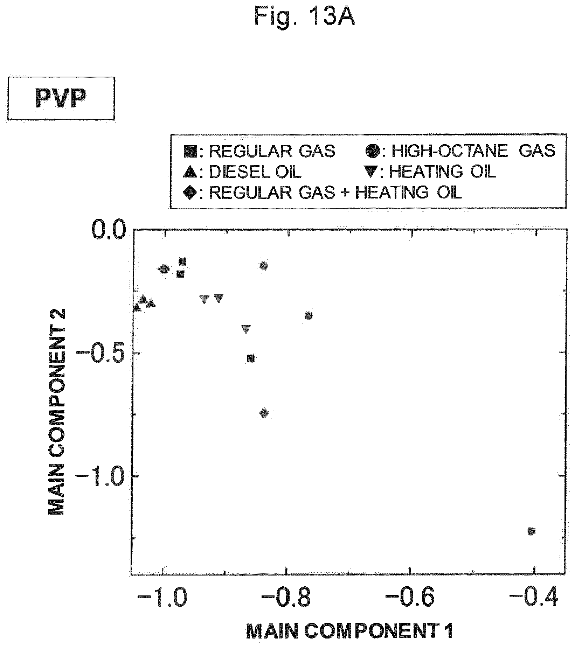

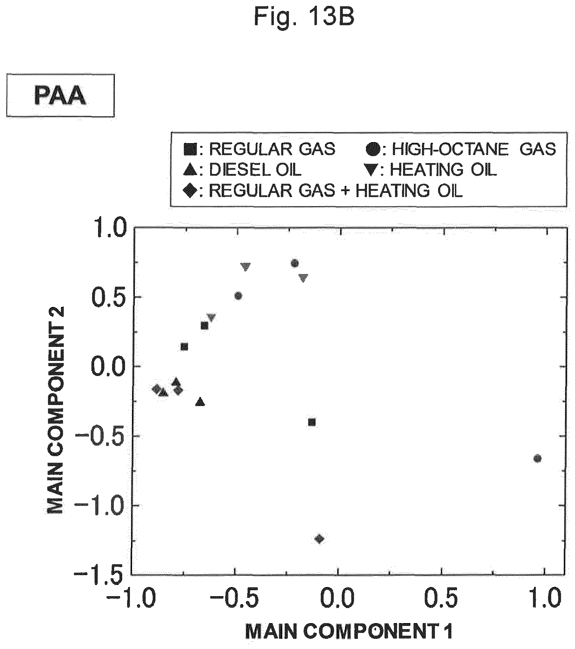

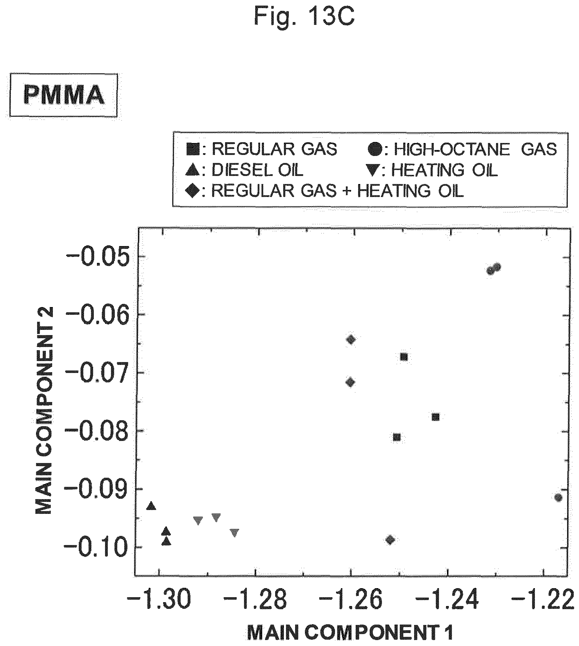

Results of principal component analysis performed according to a method similar to the method described in Example 2 are shown in Fig. 12, and Fig. 13A to Fig. 13C. The results reveal that, when octadecyl group-modified nanoparticles were used, four types of fuel oils were separated, and the fuel oils can be identified, whereas identification of the fuel oils was difficult by using the three types of polymers. The reason for this can be explained on the basis of the fact that constituents of the four types of fuel oils are slightly different from each other. That is, it is known that diesel oil and heating oil contain a high percentage of long chain alkanes, whereas regular gas and high-octane gas contain a high percentage of volatile short chain alkanes. With respect to shapes of signals, results of measurements of regular gas and high-octane gas shown in Fig. 10 are similar to shapes with respect to n-hexane, n-heptane, and n-octane shown in Fig. 6, and results of measurements of diesel oil and heating oil shown in Fig. 10 are similar to shapes with respect to n-undecane and n-dodecane shown in Fig. 6.

An octadecyl group-modified nanoparticle of the present invention can identify difference in only one carbon atom in normal alkane molecules as shown also in Example 2. Accordingly, when a main component is several types of normal alkanes, and contents of the normal alkanes are different with one another as in fuel oils used in the present examples, highly accurate identification can be achieved by using only a single receptor. Further, as can be seen from Fig. 12, since diesel oil and heating oil can be clearly identified, an adulterated diesel oil using heating oil or heavy oil and a proper diesel oil can be identified.

Further, when regular gasoline was mixed with a small amount of heating oil in a volume ratio of 9:1 and the mixture was measured, results of the measurement were different from those with respect to regular gas and heating oil, and thus it was found that identification was possible by principal component analysis. This indicates that a sample of gasoline with alcohol or heating oil mixed in for the purpose of adding volume can easily be identified by using a single receptor.

### <Example 4> A surface stress sensor coated with dodecyl group modified silica-titania hybrid nanoparticles

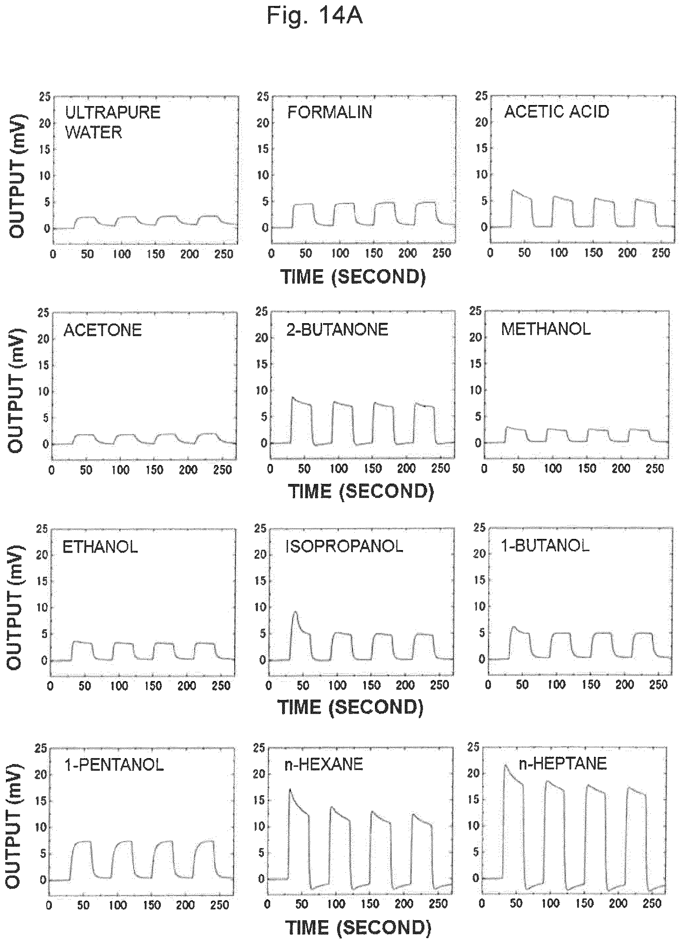

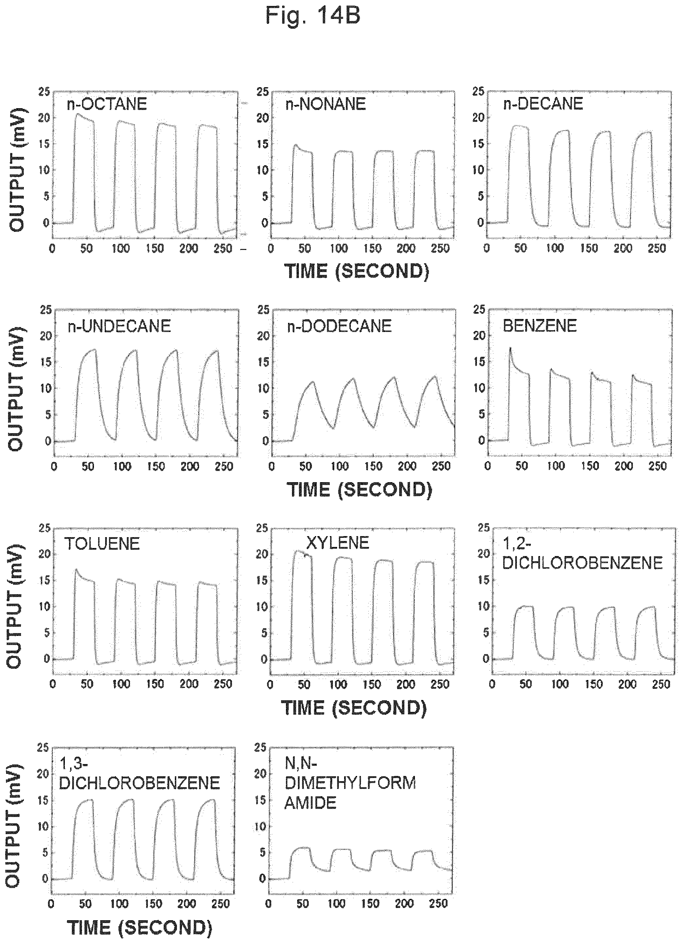

Silica-titania hybrid nanoparticles modified with dodecyl groups were synthesized according to the method described in Example 1. The only difference in the synthesis procedures is that dodecyltriethoxysilane (DDTES) was used instead of ODTES. Then, dispersions were prepared according to the method described in Example 1, and a surface of a sensor was coated by spray coating. Then, measurements of 23 types of chemical species described in Example 1 were performed. Here, sample vapors were obtained also at room temperature. Results of the measurements are shown in Fig. 14A and Fig. 14B.

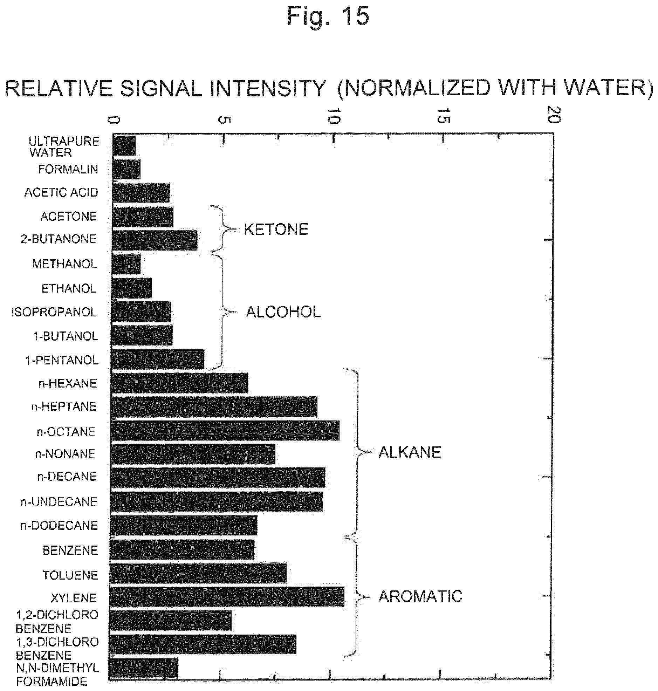

Further, relative signal intensities in which signal intensities with respect to sample vapors are divided by a signal intensity with respect to water vapor are shown in Fig. 15. Tendencies of the signal intensities are almost similar to those with respect to octadecyl group-modified nanoparticles, and responses with respect to compounds which are slightly soluble in water are larger than responses with respect to water soluble compounds. However, ratios of signal intensities obtained by measurements of the compounds which are slightly soluble in water to signal intensities obtained by measurements of the water soluble compounds are lower than ratios obtained by using octadecyl group-modified nanoparticles. The reduced ratios are thought to reflect smaller hydrophobicity by using a dodecyl group having fewer carbon atoms than those of an octadecyl group. In view of the fact that alcohol and the like is used for the purpose of increasing volume, even a single receptor can exhibit a high ability of identifying fuel oils by using nanoparticles modified with an octadecyl group, which has a large numbers of carbon atoms.

### Industrial Applicability

As described above, commonly used hydrocarbons such as fuel oils can easily be identified according to the present invention. Accordingly, without limitation, when the present invention is applied to, for example, a fuel supply system of an internal combustion engine, a fuel supplied by accident, a fuel oil deteriorated by long-term storage, and a fraudulent fuel oil (e.g., gasoline, diesel oil), which can cause damage to an internal combustion engine or adjunct devices of the engine, can easily be detected, and thus the present invention is expected to be widely applicable to industries.

### Citation List

### Patent Literature

Patent Literature 1: WO 2011/148774
Patent Literature 2: WO 2016/121155

### Non Patent Literature

Non Patent Literature 1: Huihui Guo, Xiangdong Chen, and Yao Yao, "A Room Temperature polymer-Coated Piezoresistive Silicon Bridge Gasoline Vapor Sensor," IEEE SENSORS JOURNAL, VOL. 12, NO. 5, 926-929, (2012).
Non Patent Literature 2: K. Shiba, T. Sugiyama, T. Takei, and G. Yoshikawa, "Controlled growth of silica-titania hybrid functional nanoparticles through a multistep microfluidic approach," Chem. Commun. 51, 15854-15857 (2015).
Non Patent Literature 3: H. AIQahtani, M. Sugden, D. Puzzovio, L. Hague, N. Mullin, T. Richardson, and M. Grell, "Highly sensitive alkane odour sensors based on functionalised gold nanoparticles", Sensors and Actuators, Vol. 160, Nr. 1, 399-404 (2011).

## Claims

1. A fuel oil identification method comprising:
supplying vapor of an analyte to a fuel oil identification sensor; and
identifying the analyte on the basis of an output of the fuel oil identification sensor
**characterized in that** the fuel oil identification sensor comprises:
a surface stress sensor, and
a receptor layer comprising particles of 1 mm or less in particle size and modified with hydrocarbon groups, the receptor layer coating a surface of the surface stress sensor, and
wherein said surface stress sensor is adapted to detect a change in surface stress when said receptor layer is contacted by vapor of said fuel oil.

2. A fuel oil identification method according to claim 1, wherein the particles are nanoparticles having a particle size of 100 nm or less.

3. A fuel oil identification method according to either of claims 1 and 2, wherein the particles comprise at least one member selected from the group consisting of: zeolite; a metal-organic framework; a hybrid of an oxide and/or a metal and various surfactants; a porous material; graphene; an alkali silicate or a titanate such as a clay mineral; and a low-dimensional compound comprising a transition metal oxoate.

4. A fuel oil identification method according to any of claims 1 to 3, wherein the receptor layer further comprises a matrix material other than the particles.

5. A fuel oil identification method according to any of claims 1 to 4, wherein the hydrocarbon group is an alkyl group or an aryl group.

6. A fuel oil identification method according to claim 5, wherein the alkyl group is an alkyl group having 1 to 30 carbon atoms.

7. A fuel oil identification method according to claim 6, wherein the alkyl group is a dodecyl group or an octadecyl group.

8. A fuel oil identification method according to any of claims 1 to 7, wherein the fuel oil is selected from the group consisting of regular gasoline; high-octane gasoline; diesel oil; heating oil; a mixture of regular gasoline or high-octane gasoline with heating oil, a mixture of regular gasoline or high-octane gasoline with an alcohol; heavy oil A; a mixture of heavy oil A with heating oil; and a mixture of at least one of heavy oil A and heating oil with diesel oil.

9. A fuel oil identification method according to any one of claims 1 to 8, wherein a mixture of the vapor of the analyte and another gas is supplied to the fuel oil identification sensor.

10. A fuel oil identification method according to claim 9, wherein the other gas is a gas or a mixture of a plurality of gases selected from the group consisting of nitrogen, air, argon, and helium.

11. A fuel oil identification method according to any of claims 1 to 10, wherein the vapor of the analyte is generated without heating or cooling.

12. A fuel oil identification method according to any of claims 1 to 11, wherein the vapor of the analyte is generated at room temperature.

13. A fuel oil identification method according to any of claims 1 to 12, comprising:
supplying the vapor of the analyte to a plurality of types of fuel oil identification sensors; and
identifying the analyte on the basis of outputs of the respective fuel oil identification sensors.

14. A fuel oil identification method according to any of claims 1 to 13, wherein vapors of a plurality of analytes are switched in sequence for delivery to the fuel identification sensor.

15. A fuel oil identification method according to claim 14, wherein cleaning of the fuel identification sensor is not performed for at least one cycle of the switching for the delivery.

16. A fuel oil identification method according to any of claims 1 to 15, wherein identification of the analyte is made by further performing an information processing of the output of the fuel oil identification sensor.

17. A fuel oil identification method according to claim 16, wherein the information processing is based on at least one analytical method selected from principal component analysis, a neural network, deep learning, a support vector machine, random forest, a decision tree, regression analysis, and big data analysis.

## Patentansprüche

1. Brennstoffölidentifikationsverfahren, umfassend:
Leiten von Dampf eines Analyten zu einem Brennstoffölidentifikationssensor und
Identifizieren des Analyten basierend auf einer Ausgabe des Brennstoffölidentifikationssensors,
**dadurch gekennzeichnet, dass** der Brennstoffölidentifikationssensor umfasst:
einen Oberflächenspannungssensor und
eine Rezeptorschicht, umfassend Partikel mit einer Partikelgröße von 1 mm oder weniger und mit Kohlenwasserstoffgruppen modifiziert, wobei die Rezeptorschicht eine Oberfläche des Oberflächenspannungssensors beschichtet, und
wobei der Oberflächenspannungssensor dafür konzipiert ist, eine Änderung der Oberflächenspannung zu detektieren, wenn die Rezeptorschicht durch Dampf des Brennstofföls kontaktiert wird.

2. Brennstoffölidentifikationsverfahren nach Anspruch 1, wobei die Partikel Nanopartikel sind, die eine Partikelgröße von 100 nm oder weniger aufweisen.

3. Brennstoffölidentifikationsverfahren nach einem der Ansprüche 1 und 2, wobei die Partikel mindestens ein Element umfassen, das ausgewählt ist aus der Gruppe bestehend aus: Zeolith; einem metallorganischen Gerüst; einem Hybrid eines Oxids und/oder eines Metalls und verschiedener Tenside; einem porösen Material; Graphen; einem Alkalisilikat oder einem Titanat, wie einem Tonmineral; und einer niedrigdimensionalen Verbindung, umfassend ein Übergangsmetalloxoat.

4. Brennstoffölidentifikationsverfahren nach einem der Ansprüche 1 bis 3, wobei die Rezeptorschicht weiter ein anderes Matrixmaterial als die Partikel umfasst.

5. Brennstoffölidentifikationsverfahren nach einem der Ansprüche 1 bis 4, wobei die Kohlenwasserstoffgruppe eine Alkylgruppe oder eine Arylgruppe ist.

6. Brennstoffölidentifikationsverfahren nach Anspruch 5, wobei die Alkylgruppe eine Alkylgruppe ist, die 1 bis 30 Kohlenstoffatome aufweist.

7. Brennstoffölidentifikationsverfahren nach Anspruch 6, wobei die Alkylgruppe eine Dodecylgruppe oder eine Octadecylgruppe ist.

8. Brennstoffölidentifikationsverfahren nach einem der Ansprüche 1 bis 7, wobei das Brennstofföl ausgewählt ist aus der Gruppe bestehend aus normalem Benzin; Benzin mit hoher Oktanzahl; Dieselöl; Heizöl; einem Gemisch aus normalem Benzin oder Benzin mit hoher Oktanzahl mit Heizöl, einem Gemisch aus normalem Benzin oder Benzin mit hoher Oktanzahl mit einem Alkohol; Schweröl A; einem Gemisch aus Schweröl A mit Heizöl; und einem Gemisch aus mindestens einem von Schweröl A und Heizöl mit Dieselöl.

9. Brennstoffölidentifikationsverfahren nach einem der Ansprüche 1 bis 8, wobei ein Gemisch des Dampfs des Analyten und eines anderen Gases zu dem Brennstoffölidentifikationssensor geleitet wird.

10. Brennstoffölidentifikationsverfahren nach Anspruch 9, wobei das andere Gas ein Gas oder ein Gemisch einer Vielzahl von Gasen, ausgewählt aus der Gruppe bestehend aus Stickstoff, Luft, Argon und Helium, ist.

11. Brennstoffölidentifikationsverfahren nach einem der Ansprüche 1 bis 10, wobei der Dampf des Analyten ohne Erhitzen oder Abkühlen erzeugt wird.

12. Brennstoffölidentifikationsverfahren nach einem der Ansprüche 1 bis 11, wobei der Dampf des Analyten bei Raumtemperatur erzeugt wird.

13. Brennstoffölidentifikationsverfahren nach einem der Ansprüche 1 bis 12, umfassend:
Leiten des Dampfs des Analyten zu einer Vielzahl von Typen von Brennstoffölidentifikationssensoren und
Identifizieren des Analyten basierend auf Ausgaben der jeweiligen Brennstoffölidentifikationssensoren.

14. Brennstoffölidentifikationsverfahren nach einem der Ansprüche 1 bis 13, wobei Dämpfe einer Vielzahl von Analyten der Reihe nach zur Zufuhr zu dem Kraftstoffidentifikationssensor geschaltet werden.

15. Brennstoffölidentifikationsverfahren nach Anspruch 14, wobei das Reinigen des Kraftstoffidentifikationssensors für mindestens einen Zyklus des Schaltens für die Zufuhr nicht durchgeführt wird.

16. Brennstoffölidentifikationsverfahren nach einem der Ansprüche 1 bis 15, wobei die Identifikation des Analyten durch weiteres Durchführen einer Informationsverarbeitung der Ausgabe des Brennstoffölidentifikationssensors erfolgt.

17. Brennstoffölidentifikationsverfahren nach Anspruch 16, wobei die Informationsverarbeitung auf mindestens einem analytischen Verfahren basiert, das ausgewählt ist aus einer Hauptkomponentenanalyse, einem neuronalen Netzwerk, Tiefem Lernen, einer Stützvektormaschine, einem Random Forest, einem Entscheidungsbaum, einer Regressionsanalyse und einer Massendatenanalyse.

## Revendications

1. Procédé d'identification de carburant comprenant :
la fourniture de vapeur d'un analyte à un capteur d'identification de carburant ; et
l'identification de l'analyte sur la base d'une sortie du capteur d'identification de carburant,
**caractérisé en ce que** le capteur d'identification de carburant comprend :
un capteur de contrainte de surface, et
une couche réceptrice comprenant des particules de 1 mm ou moins de taille particulaire et modifiées par des groupements hydrocarbure, la couche réceptrice revêtant une surface du capteur de contrainte de surface, et
dans lequel ledit capteur de contrainte de surface est à même de détecter un changement de contrainte de surface lorsque ladite couche réceptrice vient en contact avec la vapeur dudit carburant.

2. Procédé d'identification de carburant selon la revendication 1, dans lequel les particules sont des nanoparticules ayant une taille particulaire de 100 nm ou moins.

3. Procédé d'identification de carburant selon l'une ou l'autre des revendications 1 et 2, dans lequel les particules comprennent au moins un élément sélectionné dans le groupe consistant en : une zéolite; une ossature organométallique ; un hybride d'un oxyde et/ou d'un métal et de divers tensioactifs ; un matériau poreux ; du graphène ; un silicate alcalin ou un titanate tel qu'un minéral argileux; et un composé dimensionnellement faible comprenant un oxoate de métal de transition.

4. Procédé d'identification de carburant selon l'une quelconque des revendications 1 à 3, dans lequel la couche réceptrice comprend en outre un matériau de matrice autre que les particules.

5. Procédé d'identification de carburant selon l'une quelconque des revendications 1 à 4, dans lequel le groupement hydrocarbure est un groupement alkyle ou un groupement aryle.

6. Procédé d'identification de carburant selon la revendication 5, dans lequel le groupement alkyle est un groupement alkyle ayant 1 à 30 atomes de carbone.

7. Procédé d'identification de carburant selon la revendication 6, dans lequel le groupement alkyle est un groupement dodécyle ou un groupement octadécyle.

8. Procédé d'identification de carburant selon l'une quelconque des revendications 1 à 7, dans lequel le carburant est sélectionné dans le groupe consistant en de l'essence ordinaire; de l'essence à indice d'octane élevé; du carburant diésel ; du mazout; un mélange d'essence ordinaire ou d'essence à indice d'octane élevé avec du mazout, un mélange d'essence ordinaire ou d'essence à indice d'octane élevé avec un alcool ; du pétrole lourd A; un mélange de pétrole lourd A avec du mazout; et un mélange d'au moins l'un de pétrole lourd A et de mazout avec un carburant diésel.

9. Procédé d'identification de carburant selon l'une quelconque des revendications 1 à 8, dans lequel un mélange de la vapeur de l'analyte et d'un autre gaz est fourni au capteur d'identification de carburant.

10. Procédé d'identification de carburant selon la revendication 9, dans lequel l'autre gaz est un gaz ou un mélange d'une pluralité de gaz sélectionnés dans le groupe consistant en l'azote, l'air, l'argon et l'hélium.

11. Procédé d'identification de carburant selon l'une quelconque des revendications 1 à 10, dans lequel la vapeur de l'analyte est générée sans chauffage ni refroidissement.

12. Procédé d'identification de carburant selon l'une quelconque des revendications 1 à 11, dans lequel la vapeur de l'analyte est générée à température ambiante.

13. Procédé d'identification de carburant selon l'une quelconque des revendications 1 à 12, comprenant :
la fourniture de la vapeur de l'analyte à une pluralité de types de capteurs d'identification de carburant ; et
l'identification de l'analyte sur la base de sorties des capteurs d'identification de carburant respectifs.

14. Procédé d'identification de carburant selon l'une quelconque des revendications 1 à 13, dans lequel des vapeurs d'une pluralité d'analytes sont alternées en séquence pour la fourniture au capteur d'identification de carburant.

15. Procédé d'identification de carburant selon la revendication 14, dans lequel le nettoyage du capteur d'identification de carburant n'est pas réalisé pendant au moins un cycle de l'alternance pour la fourniture.

16. Procédé d'identification de carburant selon l'une quelconque des revendications 1 à 15, dans lequel l'identification de l'analyte est effectuée en réalisant en outre un traitement d'information de la sortie du capteur d'identification de carburant.

17. Procédé d'identification de carburant selon la revendication 16, dans lequel le traitement d'information est basé sur au moins un procédé analytique sélectionné parmi une analyse des composantes principales, un réseau neuronal, un apprentissage profond, une machine à vecteurs de support, une forêt aléatoire, un arbre de décision, une analyse de régression et une analyse des données massives.
